# EUROPEAN PATENT SPECIFICATION

(11) **EP 1 796 723 B1**
(45) Date of publication and mention of the grant of the patent: **14.11.2012**
(21) Application number: 05792228.8
(22) Date of filing: 07.10.2005
(51) Int. Cl.: A61K 39/395

(54) **METHOD OF DIAGNOSING CANCER BY DETECTING THE EXPRESSION OF SPAG9 OR THE PRESENCE OF ANTI-SPAG9 ANTIBODIES**
VERFHAREN ZUR DIAGNOSE VON KREBS DURCH NACHWEIS DER EXPRESSION VON SPAG9 ODER NACHWEIS VON ANTIKÖRPER GEGEN SAPG9
MÉTHODE DE DIAGNOSE DU CANCER EN DÉTECTANT L'EXPRESSION DE SPAG9 OU DES ANTICORPS CONTRE LE SPAG9

(30) Priority: 07.10.2004 IN DE19452004
(43) Date of publication of application: 20.06.2007
(73) Proprietor: National Institute of Immunology, New Delhi 110067 (IN)
(72) Inventor: SURI, Anil Kumar, National Inst. of Immunology, New Delhi 110 067 (IN)
(74) Representative: Cabinet Plasseraud
(86) International application number: PCT/IB2005/002972
(87) International publication number: WO 2006/038101

(56) References cited:
- WO-A-2004/030615
- SHANKAR SRINIVASAN ET AL: "Cloning of a novel human testis mRNA specifically expressed in testicular haploid germ cells, having unique palindromic sequences and encoding a leucine zipper dimerization motif" BIOCHEMICAL AND BIOPHYSICAL RESEARCH COMMUNICATIONS, vol. 243, no. 2, 13 February 1998 (1998-02-13), pages 561-565, XP002405527 ISSN: 0006-291X
- OLD LLOYD J ET AL: "New paths in human cancer serology" JOURNAL OF EXPERIMENTAL MEDICINE, TOKYO, JP, vol. 187, no. 8, 20 April 1998 (1998-04-20), pages 1163-1167, XP002193786 ISSN: 0022-1007
- WILLIS SIMON ET AL: "Detailed gene copy number and RNA expression analysis of the 17q12-23 region in primary breast cancers." GENES CHROMOSOMES AND CANCER, vol. 36, no. 4, April 2003 (2003-04), pages 382-392, XP002405528 ISSN: 1045-2257
- KITAHARA OSAMU ET AL: "Classification of sensitivity or resistance of cervical cancers to ionizing radiation according to expression profiles of 62 genes selected by cDNA microarray analysis" NEOPLASIA (NEW YORK), vol. 4, no. 4, July 2002 (2002-07), pages 295-303, XP002405529 ISSN: 1522-8002
- LINN SABINE C ET AL: "Gene expression patterns and gene copy number changes in dermatofibrosarcoma protuberans." AMERICAN JOURNAL OF PATHOLOGY, vol. 163, no. 6, December 2003 (2003-12), pages 2383-2395, XP002405530 ISSN: 0002-9440
- HELM JAMES ET AL: "Dedifferentiation precedes invasion in the progression from Barrett's metaplasia to esophageal adenocarcinoma." CLINICAL CANCER RESEARCH : AN OFFICIAL JOURNAL OF THE AMERICAN ASSOCIATION FOR CANCER RESEARCH. 1 APR 2005, vol. 11, no. 7, 1 April 2005 (2005-04-01), pages 2478-2485, XP002405531 ISSN: 1078-0432
- JAGADISH NIRMALA ET AL: "Sperm associated antigen 9 (SPAG9): a new member of c-Jun NH2-terminal kinase (JNK) interacting protein exclusively expressed in testis" KEIO JOURNAL OF MEDICINE, vol. 54, no. 2, June 2005 (2005-06), pages 66-71, XP002405532 ISSN: 0022-9717
- JAGADISH NIRMALA ET AL: "Characterization of a novel human sperm-associated antigen 9 (SPAG9) having structural homology with c-Jun N-terminal kinase-interacting protein." THE BIOCHEMICAL JOURNAL. 1 JUL 2005, vol. 389, no. Pt 1, 1 July 2005 (2005-07-01), pages 73-82, XP002405533 ISSN: 1470-8728

## Description

### FIELD OF THE INVENTION:

The present disclosure relates to novel cancer associated antibodies and antigens and their use in cancer diagnosis.

### BACKGROUND OF THE INVENTION:

Cancer, as is known is a killer disease and is a subject of investigation and research by several workers in various countries. It is observed that in most cases, cancer is diagnosed and treated only in the advanced stage, when the cancer cells have already invaded and metastasised throughout the body. More than 60% of patients with breast, lung, colon and ovarian cancer already have hidden or overt metastatic colonies. At this stage, therapeutic modalities are limited in their success. Detecting cancers in early stages even in the premalignant state, means that current or future treatment modalities might have a higher likelihood of a true cure.

Ovarian cancer is a prime example of this clinical dilemma. More than two-thirds of cases of ovarian cancer are detected at an advanced stage, when the ovarian cancer cells have spread away from the ovary- and have disseminated throughout the peritoneal cavity [Gure, A.O. Altorki, N.K. Stockert E, Scanlan M.J., Old L.J, & Chen Y.T. (1998) Cancer Res 58, 1034-1341]. Although the disease at this stage is advanced, it rarely produces specific or diagnostic symptoms. Consequently, ovarian cancer is usually treated when it is at an advanced stage. The resulting five year survival rate is 35-40% for late-stage patients who receive the best possible surgical and chemotherapeutic intervention.

By contrast, if ovarian cancer is detected when it is still confined to the ovary (stage I), conventional therapy produces a high rate (95%) of five-year survival. Hence, early detection of ovarian cancer lacks a specific symptom, a specific biomarker and accurate and reliable diagnostic, non-invasive modalities.

Traditional methods of treating cancer include use of non-invasive or mildly invasive diagnostic tests, biopsy, histological examination and so on. However, each of these tests have their own limitations and hence the search for new methods of diagnosis and treatment of cancer.

In keeping with the above, it is desirable to provide an effective, clinically useful biomarker measurable in a readily accessible body fluid and tissues. Clinical proteomics is well suited to discovery of such biomarkers, as serum is a protein-rich information reservoir that contains the traces of what has been encountered by the blood during its constant perfusion and percolation throughout the body.

However, until now, the search for cancer-related biomarkers for early disease detection has been a 'one-at-a-time' approach, which has looked for over-expressed proteins in blood that are shed into the circulation as a consequence of the disease process [Adam, B.L., Vlahou, A., Semmes, O.J. & Wright, G.L. Jr. (2001) Proteomic approaches to biomarker discovery in prostate and bladder cancers. Proteomics 1, 1264-1270; Carter, D. et al. (2002) Purification and characterization of the mammaglobin/lipophilin B complex, a promising diagnostic marker for breast cancer, Biochemistry 41, 6714-6722; Rosty, C. et al (2002) Identification of hepatocarcinoma-intestinepancreas/ pancreatitis-associated protein I as a biomarker for pancreatic ductal adenocarcinoma by protein biochip technology. Cancer Res. 62, 2868-1875; Xiao, Z. et al. (2001) Quantitation of serum prostate-specific membrane antigen by a novel protein biochip immunoassay discriminates benign from malignant prostate disease. Cancer Res. 61, 6029-6033; Kim, J.H. et al (2002) Osteopontin as a potential diagnostic biomarker for ovarian cancer. JAMA 287, 1671-1679]. Thus, the art so far has provided markers that are tissue specific and assist in identification of a specific type of cancer. None of them serve as universal markers or have universal application.

WILLIS SIMON ET AL: "Detailed gene copy number and RNA expression analysis of the 17q12-23 region in primary breast cancers." GENES CHROMOSOMES AND CANCER, vol. 36, no. 4, April 2003 (2003-04), pages 382-392, discloses that SPAG9 is part of set of 24 genes "which are potential oncogenes in *breast carcinomas* and which merit further evaluation in that context".

LINN SABINE C ET AL: "Gene expression patterns and gene copy number changes in dermatofibrosarcoma protuberans.", AMERICAN JOURNAL OF PATHOLOGY, vol. 163, no. 6, December 2003 (2003-12), pages 2383-2395, shows that SPAG9 is part of group of 259 genes "whose elevated expression was most consistently associated with a diagnosis of *DFPS*".

KITAHARA OSAMU ET AL: "Classification of sensitivity or resistance of cervical cancers to ionizing radiation according to expression profiles of 62 genes selected by cDNA microarray analysis",NEOPLASIA (NEW YORK), vol. 4, no. 4, July 2002 (2002-07), pages 295-303 reveals that SPAG9 belongs to a group of 121 genes whose expression is increased in radiosensitive *cervical squamous cell carcinoma.*

Hence, there is a need in art to develop diagnostic agents that may be used as markers having universal application and thus useful for detection of any type of cancer. To fulfil this need, the invention provides novel antibodies against SEQ ID NO:2, its isoform or a polypeptide comprising SEQ ID NO:2 and its use in diagnosis of cancer.

### OBJECTS OF THE INVENTION:

The main object of the invention is to provide methods for detection of cancer according to the appended claims.

### DESCRIPTION OF THE INVENTION:

The inventors during some of their investigations had found a novel gene encoding sperm associated antigen 9 (SPAG9) [labelled herein as SEQ ID NO: 1] which has been cloned from human testis cDNA library. The 2523 bp cDNA ([Shankar, Mohapatra and Suri (1998) Biochem. Biophys. Res. Commun. 243, 561-565; Acc. No: X91879] contains five direct repeats, three mirror repeats, three possible stem loop structures and three palindromic motifs. Open reading frame encodes a protein of 766 amino acids. The deduced protein analysis revealed several features: 1) a characteristic leucine zipper motif (LZ); 2) an extended coiled-coil domain (coil) and 3) a transmembrane domain (T). The amino acid sequence of SPAG9 (labelled herein as SEQ ID NO:2) further revealed that primary sequence identity to JNK binding domain. The secondary structural analysis revealed that SEQ ID NO:2 has an α-helical structure. CD spectra analysis further supported a predominant α-helical structure of SEQ ID NO:2. Microsequencing of oligomeric aggregates of recombinant SEQ ID NO:2 by tandem mass spectrometry confirmed the amino acid sequence and mono atomic mass to 83.9 kDa. The studies also revealed that SEQ ID NO: 1 and 2 play a role in sperm-egg interaction. The said SEQ ID NO: 1 was reported as a member of a new family of testis specific genes with a specific function and considered as a possible contraceptive target.

Thereafter, while the inventor was conducting certain unrelated studies on the MAPK (mitogen activated protein kinases pathway) and the involvement of proteins in this pathway, the inventor surprisingly found that SEQ ID NO:2 interacted with JNK signalling pathway. In fact, it had higher binding affinity to JNK3 and JNK2 compared with JNK1. No interaction was observed with p38α or ERK pathways. The said MAPK studies suggested that SEQ ID NO:2 molecule may be involved in cell-signalling pathway supporting cellular growth and cellular proliferation. This lead the inventor to investigate further the role of SEQ ID NO:2 in cancerous tissues as testis also involves a continuous proliferation of cells, almost mimicking the cancerous cell growth.

Surprisingly, the inventor found SEQ ID NO:1 nucleotide homology with various cancer tissue ESTs. In fact when tissue distribution of SEQ ID NO: 1 was studied using different tissues, the inventors further found that mRNA of SEQ ID NO: 1 is expressed exclusively in normal testis tissue. However, biological samples from various cancer patients revealed the presence of SEQ ID NO:2 in cancerous tissues such as lung, breast, stomach, uterus, esophagus, colon, ovarian, testis, cervix, skin, prostate, oral, bladder, endometrial, kidney, liver, brain, blood, vulva, vagina, gall bladder, eye and bone.

While not wishing to be bound by any theory, the inventor believes that SEQ ID NO:2 polypeptide peptide expressed in the aforesaid cancerous tissues may be recognized as a non-self protein by the immune system and may mount an auto-antibody response in such patients resulting in anti-SEQ ID NO:2 antibodies circulating in the blood stream of cancer patients. Such antibodies may provide a basis for early detection of cancer.

### Anti-SEQ ID NO:2 antibodies:

Accordingly, in one aspect, the disclosure relates to a novel antibody or an antigen-binding fragment thereof, having specificity for a polypeptide bearing SEQ ID No:2, or an isoform thereof or a polypeptide comprising the said SEQ ID NO:2. The said antibody may be an IgG antibody or IgM antibody or a single chain or a fragment of the said antibody having specificity to the said polypeptide of SEQ ID NO:2.

Such antibodies serve as markers as they appear to be found in subjects suffering from cancer. The said antibodies of the invention are termed as "anti-SEQ ID No:2 antibody" and referred to as such hereafter. The anti-SEQ ID No:2 antibody as referred includes the antigen-binding fragment thereof. The term "antibody", as used herein includes a polyclonal antibody, a monoclonal antibody, a humanized antibody and a single chain antibody.

The disclosure also relates to a method for production of polyclonal anti-SEQ ID No:2 antibody comprising the steps of:
- providing purified polypeptide of SEQ ID NO: 2 or synthesizing the same recombinantly by cloning and expressing SEQ ID NO: 1 nucleic acid sequence in a suitable host;
- mixing SEQ ID NO:2 with an adjuvant to obtain a composition,
- injecting the composition into an animal to as produce an immune response, and
- collecting the sera from the animal and isolating polyclonal antibody generated from the same.

In the method described above, the sera from the animal may be used directly or purified prior to use by various methods including affinity chromatography employing Protein A-Sepharose, antigen Sepharose or anti-mouse-Ig-Sepharose.

### Antibody detection kit:

In another aspect, the disclosure relates to a kit useful for detection of cancer in a biological sample of a subject suspected of or suffering from cancer. The said kit comprises a polypeptide sequence of SEQ ID NO:2 or an isoform thereof or a polypeptide comprising the said SEQ ID NO:2 as described herein above coupled to a solid matrix and instructional material. The solid matrix as referred herein may include nitrocellulose paper, glass slide, microtitre plates and wells.

In yet another aspect, the invention provides method according to claim 1 for detecting a cancer in a subject suffering from or suspected of suffering from a cancer, comprising the steps of:
- providing a biological sample of a subject suspected of cancer; and
- detecting the presence of antibodies generated against SEQ ID NO:2 or an isoform thereof or a polypeptide comprising SEQ ID NO:2 in the sample,
wherein presence of antibodies generated against SEQ ID NO:2 or an isofonn thereof or a polypeptide comprising the said SEQ ID NO:2 is indicative of cancer.

In another aspect, the disclosure relates to a method for detecting a cancer in a subject suffering from or suspected of suffering from a cancer, comprising the steps of:
- providing a biological sample of a subject suspected of cancer;
- contacting the sample with the kit as set forth above and determining binding of SEQ ID NO:2 or an isoform thereof or a polypeptide comprising the said SEQ ID NO:2 with anti-SEQ ID No:2 antibody if present in biological sample through a detectable signal, which is indicative of cancer.

The detectable signal as referred herein may be any detectable signal such as development of color or fluorescence. For example, a secondary labelled antibody (enzyme conjugated antibody) may be introduced which may bind to anti- SEQ ID No:2 antibody if present in patient's sample and provide a detectable signal which may be used as a parameter for detection of cancer.

### Antigen-detection kit:

The antigen detection kit comprises an antibody having specificity to a polypeptide sequence bearing SEQ ID NO:2 or an isoform thereof or a polypeptide comprising SEQ ID NO:2. The kit is useful for detection of cancer.

In yet another aspect, the invention provides method according to claim 6 for detecting a cancer in a subject suffering from or suspected of suffering from a cancer, comprising the steps of:
- providing a biological tissue of a subject suspected of cancer; and
- detecting the presence of sequence of SEQ ID NO:2 polypeptide or an isoform thereof or a polypeptide comprising SEQ ID NO:2 in the tissue,
the presence of which is indicative of cancer.

In another aspect, the disclosed relates to a method for detecting a cancer in a subject suffering from or suspected of suffering from a cancer, comprising the steps of:
- providing a biological tissue of a subject suspected of cancer;
- contacting the tissue with the antigen-detecting kit as set forth above and determining binding of polypeptide sequence bearing SEQ ID NO:2 [or an isoform thereof or a polypeptide comprising SEQ ID NO:2] with anti-SEQ ID No:2 antibody through a detectable signal, which is indicative of cancer.

The detectable signal as referred herein may be any detectable signal such as development of color or fluorescence. For example, a secondary labelled antibody (enzyme conjugated antibody) may be introduced which may bind to the to the anti-SEQ ID No:2 antibody if present in patient's sample and provide a detectable signal which may be used as a parameter for detection of cancer. Yet another example is where the anti-SEQ ID No:2 antibody employed in the said kit may itself be a labelled antibody (labelled for color or fluorescence) and detectable signal produced may be used as a parameter for detection of cancer.

The term 'biological sample' as used herein refers to fluids or tissues obtained from subjects suspected of or suffering from cancer. Examples include blood, serum, plasma, tissue sample, urine and saliva. Further, 'cancer' as used herein refers to cancer of ectodermal, endodermal or mesodermal origin. The said cancer may be any cancer such as that of lung, breast, stomach, uterus, esophagus, colon, ovarian, testis, cervix, skin, prostate, oral, bladder, endometrial, kidney, liver, brain, blood, vulva, vagina, gall bladder, eye and bone. The 'subject' as referred above includes any mammal.

A few embodiments of the invention are now illustrated by the following examples, and drawings:

### BRIEF DESCRIPTION OF THE ACCOMPANYING DRAWINGS

**Figure 1** is a Dot Blot analysis comparing a control with patient's samples.
**Figure 2** which is a Western Blot analysis comparing a control with patient's samples.
**Figure 3** is the ELISA assay which indicates antibody titres in cancer patient's and healthy biological samples.
**Figure 4** represents the immunohistochemical analysis comparing the tissues obtained from normal healthy control and different cancer patients.

The examples below are meant for illustration only and are not intended as limitations on the inventive scope thereof. Various adaptations and variations that are possible in keeping with the inventive concept of the invention are deemed to fall within the scope of the invention.

### Example 1: Antibody detection kit - preparation of recombinant SEQ ID NO:2 protein

The applicant conducted several studies based on the findings of the invention and have now identified the SEQ ID NO:2 polypeptide and which cloned in a suitable vector for large scale expression and purification as under:

### Antigen preparation: Plasmid construction

Recombinant SEQ ID NO:2 protein (complete open reading frame encoding 766 amino acids) was successfully cloned in pET 28 b(+) expression vector. Briefly, a cDNA encoding a complete open reading frame of SEQ ID NO: was amplified using suitable primers. The product was digested with restriction enzymes and was inserted into digested pET28b(+) vector (Novagen, Madison, USA) containing multiple cloning sites to obtain pET28b- SEQ ID NO:2.

### Expression of SEQ ID NO:2 protein in Escherichia coli

Competent cells were prepared and transformed with pET28b- SEQ ID NO:2. Transformed cells were used to inoculate LB media for growing *E. coli* culture. Expression of recombinant SEQ ID NO:2 was induced with IPTG (isopropyl-β-D-thiogalactopyranoside) and subsequently purified.

### Purification of SEQ ID NO:2 expressed in Escherichia coli

*E. coli* culture expressing SEQ ID NO:2 was harvested, washed and sonicated using sonication buffer containing urea. The sonicated sup was loaded on to the agarose beads column charged with nickel sulphate. The column was washed with washing buffers and elutes were collected using elution buffer. The purified SEQ ID NO:2 recombinant protein analysed for amino acid sequence by Mass Spectrometry. Amino acid sequencing analysis revealed that the recombinant SEQ ID NO:2 protein was expressed as deduced amino acid sequence published (EMBL Acc. No. 91879, Shankar et al., 1998).

### Antibody-detection kit:

An antibody detection kit comprising SEQ ID NO:2 bound to a solid matrix is prepared as under:
A drop of purified SEQ ID NO:2 protein was placed on to the nitrocellulose membrane, fixed with methanol and air dried. The dipstick was dipped into transfer buffer and then air dried again. Dot blot analysis was performed by using dipsticks, which are made of polyester backing material with a nitrocellulose membrane.

To determine anti-SEQ ID No:2 antibody in the various biological samples from different cancer patients, the strip coated with SEQ ID NO:2 was dipped into the patient's biological fluid such as serum, followed by anti-human HRPO as secondary antibody (which was obtained from a commercial source). The dipstick was taken out and washed with PBS buffer. Finally, the strip was treated with 0.05% 3, 3'-Diaminobenzidene (DAB) (Sigma).

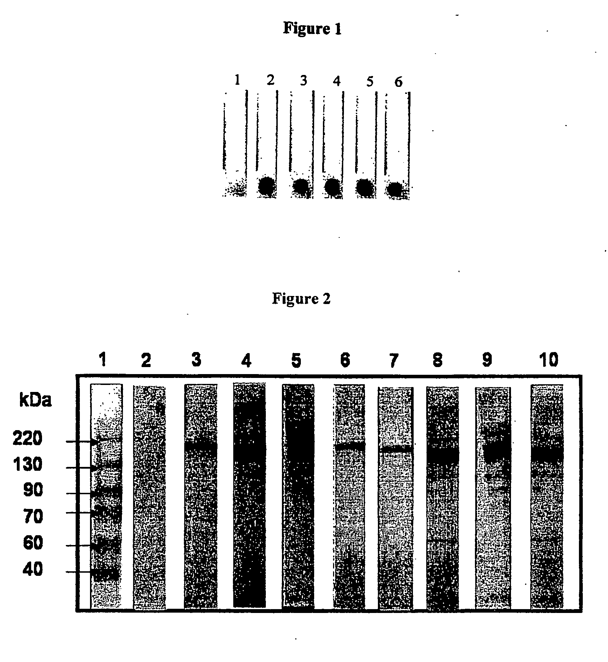

In the dot blot analysis, the appearance of a dark brown reaction on nitrocellulose membrane indicated presence of anti-SEQ ID No:2 antibody in the sample. As compared to the samples obtained from cancer pateints, the samples obtained from normal human subjects did not show any reaction. The results are shown in figure 1. As shown in figure 1, biological samples from cancer patients exhibit strong reaction as compared to a healthy subject. Lane 1 represents the reaction with serum from normal subject- there is no development of any color.. Lane 2 represents reaction with serum of cervix cancer, Lane 3 indicates reactivity of bladder cancer patient's serum, Lane 4 depicts the reaction of serum obtained from colon cancer patient, Lane 5 demonstrates the reaction of breast cancer serum, and Lane 6 is the reactivity of serum from ovarian cancer. Development of dark brown colour as in lanes 2 to 6 is indicative of cancer.

### Gel electrophoresis and Western blotting

The presence of anti-SEQ ID No:2 antibodies in a biological sample may also be detected by western blotting procedure wherein recombinant protein is run on SDS PAGE and transferred onto nitrocellulose matrix:
Recombinant SEQ ID NO:2 protein was run on SDS polyacrylamide gel. Briefly, the protein solution was diluted with sample buffer. The samples were then loaded onto polyacrylamide gel. After electrophoresis, proteins were transferred onto nitrocellulose membrane. Blocked membrane was probed with cancer patients' serum and subsequently with anti-human HRPO (which was obtained from a commercial source) as secondary antibody. Finally, strip was treated with 0.05% DAB.

Western blot analysis of sample of a patient suffering from cancer demonstrated a strong reactivity between cancerous sample and recombinant SEQ ID NO:2 protein. A strong band of recombinant SEQ ID NO:2 was observed on the nitrocellulose membrane strips treated with cancer patients' sample, whereas sample from normal human subjects failed to show any reactivity. The results are depicted in figure 2 which is a western blot analysis comparing a control with patient's samples. As shown in figure 2, lane 1 represents molecular weight marker, lane 2 represents sample obtained from normal person, lane 3 is the sample obtained from a subject with oral cancer, Lane 4- bladder cancer, lane 5 lung cancer, lane 6 prostrate cancer, lane 7 uterus, lane 8 ovary, lane 9 colon cancer and lane 10 breast cancer. As may be seen in the Western blot analysis, lanes 3 to 10 show presence of a band of 170 kDa which is absent in lane 2 of normal person.

### Enzyme linked immunosorbent assay (ELISA)

Further, the presence of anti-SPAG9 antibodies in a biological sample may also be detected by ELISA Technique as under:
Microtitration plates (Nunc, Rosaklide, Denmark) were coated with recombinant SEQ ID NO:2 protein for ELISA assay. Post blocking, the plates were incubated with the samples from cancer patients' and/or healthy control. Bound antibodies were revealed with anti-human immunoglobulins conjugated to horseradish peroxidase (which was obtained from a commercial source). Enzyme activation was carried out with 0.05% orthophenylenediamine as the substrate. The antibody response of cancer patients and healthy control individuals was represented as the mean of the absorbance of the sample of the individuals.

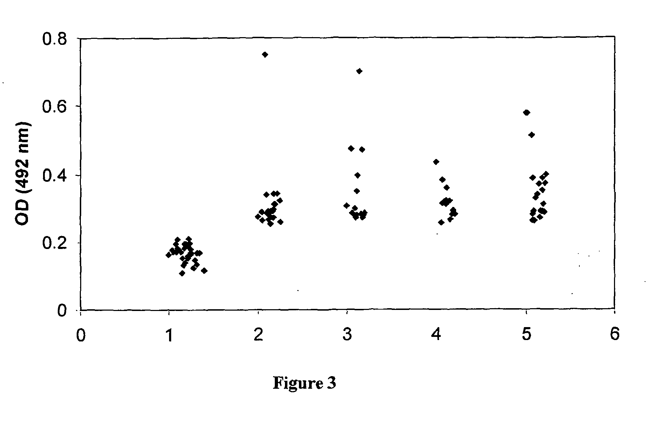

Figure 3 is the ELISA assay which indicates antibody titres in cancer patient's and healthy biological samples. The absorbance values are indicated on Y-axis whereas different cancer types are depicted as numerals on the X-axis. (1) represents the titres obtained from serum samples from normal subject, (2) represents the titres obtained from cervix cancer patients, (3) denotes the titre values from patients suffering from bladder cancer, (4) gastrointestinal tract cancer, (5) ovarian cancer. The test sample titre values are considered indicative of cancer when the estimated ELISA titres are above the mean+2SD of healthy biological sample. Cancer patient's biological sample revealed higher ELISA titre values above the mean+2SD of healthy sample as indicated in the figure, which is indicative of cancer (+2SD is the standard cut off).

**Table 1: Samples tested & Results:**

| **Biological sample** | **Dot blot analysis** | | **Western blot analysis** | | **ELISA** | |
|---|---|---|---|---|---|---|
| | **Normal** | **cancerous** | **Normal** | **cancerous** | **Normal** | **cancerous** |
| Lung | - | + | - | + | - | + |
| Breast | - | + | - | + | - | + |
| Prostrate | - | + | - | + | - | + |
| Bladder | - | + | - | + | - | + |
| Utreus | - | + | - | + | - | + |
| Esophagus | - | + | - | + | - | + |
| Testis | - | + | - | + | - | + |
| Vulva | - | + | - | + | - | + |
| Liver | - | + | - | + | - | + |
| Eye | - | + | - | + | - | + |
| Oral | - | + | - | + | - | + |
| Kidney | - | + | - | + | - | + |
| Colon | - | + | - | + | - | + |
| Cervix | - | + | - | + | - | + |
| Vagina | - | + | - | + | - | + |
| Brain | - | + | - | + | - | + |
| Blood | - | + | - | + | - | + |
| Bone | - | + | - | + | - | + |
| Stomach | - | + | - | + | - | + |
| Ovarian | - | + | - | + | - | + |
| Skin | - | + | - | + | - | + |
| Gall bladder | - | + | - | + | - | + |

### Example 2: Antigen detection kit:

### Preparation of anti-SEQ ID No:2 antibodies: Immunization of rats with recombinant SEQ ID NO:2 protein

For generating anti- SEQ ID NO:2 antibodies, adult female rats were employed. Rats were immunized with SEQ ID NO:2 protein with a primary and two booster injections at different time intervals. First immunization of was done with SEQ ID NO:2 mixed with an adjuvant. Following booster injections were carried out at weekly intervals, rats are then bled and the sera isolated.

### Purification of anti-SEQ ID No:2 antibodies from rat sera

The sera can be used directly or purified prior to use by various methods including affinity chromatography employing protein beads. Column containing protein A beads was loaded with rat sera and kept on the shaker for overnight. The column was centrifuged and flow through was collected. Subsequently, column was washed with binding buffer and finally elutes were collected after centrifugation. The concentration of antibodies was calculated by taking OD at 280 nm.

Thus the antigen detection kit was prepared comprising anti-SEQ ID No:2 antibodies, for detection of SEQ ID NO:2 protein in tissues: The details of the immunohistochemistry analysis performed is as below:
Tissues from cancerous patients were fixed by standard fixative using standard Immunohistochemistry procedure. Biological tissue sections were incubated with rat anti-SEQ ID No:2 antibody and then treated with goat anti-rat immunoglobulins conjugated to horseradish peroxidases (which was obtained from a commercial source). After washing, sections were treated with 0.01% DAB and were counter-stained with Mayer's hematoxylin.

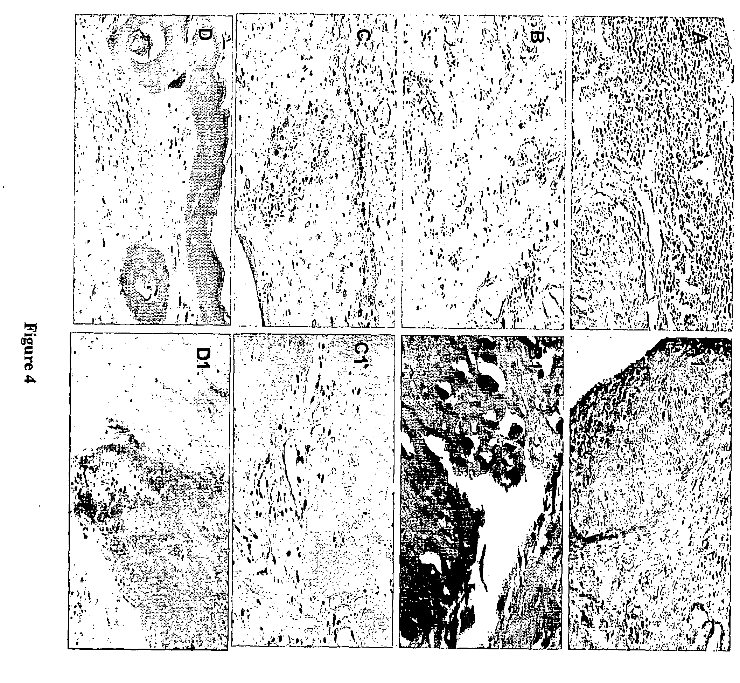

Figure 4 represents the immunohistochemical analysis comparing the tissues obtained from normal healthy control and different cancer patients. The normal tissues are in the left lane while the cancerous tissues are depicted on right side.
(A) is the immunohistochemical analysis of cervix tissue obtained from normal subject, (A1) is the tissue obtained from subject suffering form cervix cancer; (B) is immunohistochemical analysis of normal mesenchymal tissue and (B1) is the tissue obtained from the subject suffering from mesenchymal cancer; (C) is the normal tongue tissue and (C1) is the tongue cancerous tissue; (D) is the normal skin tissue and (D1) is the skin cancerous tissue;

A strong reactivity between SEQ ID NO:2 and the anti-SEQ ID No:2. antibody was observed in cancer tissues whereas tissues from normal healthy controls did not reveal any reactivity. The development of brown color in the cancer tissues is indicative of cancer in the given subject.

### Samples tested:

Several cancerous tissues were examined for the expression of SEQ ID NO:2 protein and the results are set out below:

| **Tissue type** | **Antigen detection Kit** | |
|---|---|---|
| | Normal | cancerous |
| **Lung** | - | + |
| **Breast** | - | + |
| **Prostrate** | - | + |
| **Bladder** | - | + |
| **Utreus** | - | + |
| **Esophagus** | - | + |
| **Testis** | + | + |
| **Vulva** | - | + |
| **Liver** | - | + |
| **Eye** | - | + |
| **Oral** | - | + |
| **Kidney** | - | + |
| **Colon** | - | + |
| **Cervix** | - | + |
| **Vagina** | - | + |
| **Brain** | - | + |
| **Blood** | - | + |
| **Bone** | - | + |
| **Stomach** | - | + |
| **Ovarian** | - | + |
| **Skin** | - | + |
| **Gall bladder** | - | + |

The strong reactivity of anti-SEQ ID No:2 antibodies with the cancerous tissues demonstrated the expression of SEQ ID NO:2 in various cancer cells. Since SEQ ID NO:2 is exclusively expressed in the normal testis, the presence of SEQ ID NO:2 in the tissues other than testis accounts for the onset /development of malignancy in the tissues.
**SEQ ID NO:1 Nucleotide sequence** 
1 gacaacttga gctgaaagcg aaaaactatg ctgaccagat tagcagactt gaagaaagag
61 aagcagaact gaagaaggaa tataatgcat tacatcaaag acacactgag atgatccata
121 attatatgga acatttagaa agaacaaaac ttcatcagct ctcagggagt gatcaactag
181 aatccacagc tcatagtaga attagaaaag aacgccctat atcattagga attttcccat
241 tacctgctgg agatggattg cttacacctg atgctcagaa aggaggagag acccctggat
301 ctgagcaatg gaaatttcag gaattaagtc aaccacgttc tcataccagc ctgaaggatg
361 agctttctga tgttagccaa ggcggatcta aagctaccac tccagcatca acagctaatt
421 cagatgtggc aacaattcct actgatactc ccttaaagga agaaaacgaa ggatttgtga
481 aggttacaga tgcgccaaat aaatcagaga taagcaaaca cattgaagta caggtagccc
541 aggaaactag aaatgtatct actggctctg ctgaaaatga agaaaagtca gaagttcaag
601 caatcatcga atctactcct gagctggata tggacaaaga tctcagtgga tataaaggtt
661 caagcactcc caccaaaggc atagagaaca aagcttttga tcgcaataca gaatctctct
721 ttgaagaact gtcttcagct ggctcaggcc taataggaga tgtggatgaa ggagcagatt
781 tactaggaat gggtcgggaa gttgagaatc ttatattaga aaatacacaa ctgttggaaa
841 ccaaaaatgc tttgaacata gtgaagaatg atttgatagc aaaagtggat gaactgacct
901 gtgagaaaga tgtgctgcaa ggggaattgg aggctgtgaa gcaagccaaa ctgaaactag
961 aggaaaagaa cagagaattg gaggaagagc ttaggaaagc tcgggcagaa gctgaagatg
1021 caaggcaaaa agcaaaagat gacgatgata gtgatattcc cacagcccag aggaaacggt
1081 ttactagagt agaaatggcc cgtgttctca tggagcgaaa ccagtataaa gagagattga
1141 tggagcttca ggaagctgtt cgatggacag agatgattcg ggcatcacga gaaaatccag
1201 ccatgcagga aaaaaaaagg tcaagcattt ggcagttttt cagccgactt ttcagctcct
1261 caagtaacac gactaagaag cctgaaccac ctgttaatct gaagtacaat gcacccacgt
1321 ctcatgttac tccgtccgtc aagaaaagaa gcagcacctt atctcagctc cctggggata
1381 agtccaaagc ctttgatttc cttagtgaag aaactgaagc tagtttagcc tcacgcagag
1441 aacaaaagag agagcagtat cgtcaggtaa aagcacatgt tcagaaggaa gacggtagag
1501 tgcaggcttt tggctggagt ctgcctcaga agtacaaaca ggtaaccaat ggtcaaggtg
1561 aaaataagat gaaaaattta cctgtgcctg tctatctcag acctctggat aaaaaagata
1621 catcaatgaa gctgtggtgt gctgttggag tcaatttatc tggtgggaag accagagatg
1681 gtggttctgt tgttggagca agtgtatttt acaaggatgt tgctggtttg gatacagaag
1741 gcagtaaaca gcgaagtgcc tctcagagta gtttagataa gttagatcag gaacttaagg
1801 aacagcagaa ggagttaaaa aatcaagaag aattatccag tctagtttgg atctgtacca
1861 gcactcattc ggctacaaaa gttcttatta ttgatgctgt tcaacctggc aacatcctag
1921 acagtttcac tgtttgcaac tctcatgttc tgtgcattgc aagtgtgcca ggtgcacgag
1981 aaacagacta ccctgcagga gaagatcttt cagaatctgg tcaggtagac aaagcatctt
2041 tatgtggaag tatgacaagc aacagctcag cagagacaga cagcctgtta ggaggcatca
2101 cagtggttgg ttgttctgca gaaggtgtga cgggagctgc cacttcccct agtacaaatg
2161 gtgcttctcc agtgatggat aaaccaccag aaatggaagc agaaaatagt gaggttgatg
2221 aaaatgttcc aacagcagaa gaagcaactg aagctacaga agggaatgcg gggtcagctg
2281 aagacacagt ggacatctcc caaactggcg tctacacaga gcatgtcttt acagatcctt
2341 tgggagttca gatcccagaa gacctctccc cagtgtatca gtcgaggtat aataatgggt
2401 catcaactta ggcaactggg tgaagtgcac aaataaaagg aaccaaaata atctcaaaca
2461 gtcaaaagat ttccactaat tagtgtcact gtggtggatt aaaagatacc tattcttgtg
2521 ata
**SEQ ID NO:2 AMINO ACID SEQUNCE**

1 mihnymehle rtklhqlsgs dqlestahsr irkerpislg ifplpagdgl ltpdaqkgge
61 tpgseqwkfq elsqprshts lkdelsdvsq ggskattpas tansdvatip tdtplkeene
121 gfvkvtdapn kseiskhiev qvaqetrnvs tgsaeneeks evqaiiestp eldmdkdlsg
181 ykgsstptkg ienkafdrnt eslfeelssa gsgligdvde gadllgmgre venlilentq
241 lletknalni vkndliakvd eltcekdvlq geleavkqak lkleeknrel eeelrkarae
301 aedarqkakd dddsdiptaq rkrftrvema rvlmernqyk erlmelqeav rwtemirasr
361 enpamqekkr ssiwqffsrl fssssnttkk peppvnlkyn aptshvtpsv kkrsstlsql
421 pgdkskafdf lseeteasla srreqkreqy rqvkahvqke dgrvqafgws lpqkykqvtn
481 gqgenkmknl pvpvylrpld kkdtsmklwc avgvnlsggk trdggsvvga svfykdvagl
541 dtegskqrsa sqssldkldq elkeqqkelk nqeelsslvw ictsthsatk vliidavqpg
601 nildsftvcn shvlciasvp garetdypag edlsesgqvd kaslcgsmts nssaetdsll
661 ggitvvgcsa egvtgaatsp stngaspvmd kppemeaens evdenvptae eateategna
721 gsaedtvdis qtgvytehvf tdplgvqipe dlspvyqsry nngsst

## Claims

1. A method for detecting cancer in a subject suffering from or suspected of suffering from cancer, comprising the step of detecting the presence, in a biological sample obtained from a subject suspected of cancer, of an antibody generated against a polypeptide having the amino acid sequence of SEQ ID NO:2 or an isoform thereof or of an antibody generated against a polypeptide comprising the amino acid sequence of SEQ ID NO:2, the presence of said antibodies being indicative of cancer,
wherein said cancer is selected from the group consisting of lung, stomach, oesophagus, colon, ovarian, testis, prostate, oral, bladder, liver, kidney, brain, blood, vulva, vagina, gall bladder, eye and bone cancer.

2. A method as claimed in claim 1, comprising the steps of:
- contacting said biological sample with a polypeptide having the amino acid sequence of SEQ ID NO:2 or an isoform thereof or a polypeptide comprising the amino acid sequence of SEQ ID NO:2,
- determining binding of said polypeptide having the amino acid sequence of SEQ ID NO:2 or said isoform thereof or said polypeptide comprising the amino acid sequence of SEQ ID NO:2 with an antibody through a detectable signal which is indicative of cancer.

3. A method as claimed in claim 2, wherein said polypeptide having the amino acid sequence of SEQ ID NO:2 or said isoform thereof or said polypeptide comprising the amino acid sequence of SEQ ID NO:2 is coupled to a solid matrix.

4. A method as claimed in claim 3, wherein said solid matrix comprises nitrocellulose paper, glass slide, microtitre plates or wells.

5. A method as claimed in anyone of claims 1-4, wherein the biological sample is serum obtained from a subject suspected of cancer.

6. A method for detecting cancer in a subject suffering from or suspected of suffering from cancer, comprising the step of detecting, in a biological sample obtained from a subject suspected of cancer, the presence of a polypeptide having the amino acid sequence of SEQ ID NO:2 or an isoform thereof or a polypeptide comprising the amino acid sequence of SEQ ID NO:2,
wherein said cancer is selected from the group consisting of lung, stomach, oesophagus, colon, ovarian, testis, prostate, oral, bladder, liver, kidney, brain, blood, vulva, vagina, gall bladder, eye and bone cancer.

7. A method as claimed in claim 6, comprising the steps of:
- contacting said biological sample with an antibody or a fragment thereof having specificity for a polypeptide having the amino acid sequence of SEQ ID NO:2 or an isoform thereof or a polypeptide comprising the amino acid sequence of SEQ ID NO:2, and
- determining the binding of said antibody or fragment thereof with a polypeptide having the amino acid sequence of SEQ ID NO:2 or an isoform thereof or a polypeptide comprising the amino acid sequence of SEQ ID NO:2 through a detectable signal which is indicative of cancer.

8. A method as claimed in claim 7, wherein said antibody is a monoclonal antibody, a polyclonal antibody, a humanised or a recombinant antibody.

9. A method as claimed in claim 7, wherein said antibody is a labelled antibody.

10. A method as claimed in anyone of claims 6-9, wherein the biological sample is a suspected cancerous tissue obtained from a subject suspected of cancer.

11. A method as claimed in anyone of claims 1-10, wherein the subject is a mammal.

## Patentansprüche

1. Verfahren zum Nachweis von Krebs in einer Testperson mit einem Krebsleiden oder bei der ein Krebsleiden vermutet wird, umfassend die Schritte des Nachweises der Anwesenheit eines Antikörpers in einer einer Testperson, bei der Krebs vermutet wird, entnommenen biologischen Probe, wobei der Antikörper gegen ein Polypeptid mit der Aminosäuresequenz der SEQ ID NO:2 oder einer Isoform davon erzeugt wurde, oder der Antikörper gegen ein Polypeptid umfassend die Aminosäuresequenz SEQ ID NO:2 erzeugt wurde, wobei die Anwesenheit der Antikörper auf Krebs hinweist,
wobei der Krebs ausgewählt ist aus der Gruppe bestehend aus Lungen-, Magen-, Speiseröhren-, Dickdarm-, Eierstock-, Hoden-, Prostata-, Mund-, Blase-, Leber-, Nieren-, Gehirn-, Blut-, Vulva-, Vagina-, Gallenblasen-, Augen- und Knochenkrebs.

2. Verfahren nach Anspruch 1, umfassend die Schritte:
- Kontaktieren der biologischen Probe mit einem Polypeptid mit der Aminosäuresequenz SEQ ID NO:2 oder einer Isoform davon oder einem Polypeptid umfassend die Aminosäuresequenz SEQ ID NO:2,
- Nachweis des Bindens des Polypeptids mit der Aminosäuresequenz SEQ ID NO:2 oder deren Isoform oder des Polypeptids umfassend die Aminosäuresequenz SEQ ID NO:2 mit einem Antikörper durch ein detektierbares Signal, welches auf Krebs hinweist.

3. Verfahren nach Anspruch 2, wobei das Polypeptid mit der Aminosäuresequenz SEQ ID NO:2 oder deren Isoform oder das Polypeptid umfassend die Aminosäuresequenz SEQ ID NO:2 an eine feste Matrix gekoppelt ist.

4. Verfahren nach Anspruch 3, wobei die feste Matrix Nitrozellulosepapier, Glasscheiben, Mikrotiterplatten oder Vertiefungen umfasst.

5. Verfahren nach einem der Ansprüche 1 bis 4, wobei die biologische Probe ein Serum ist, das von einer Testperson erhalten wird, bei der Krebs vermutet wird.

6. Verfahren zum Nachweis von Krebs in einer Testperson mit einem Krebsleiden oder bei der ein Krebsleiden vermutet wird, umfassend die Schritte des Nachweises der Anwesenheit eines Polypeptide in einer einer Testperson, bei der Krebs vermutet wird, entnommenen biologischen Probe, wobei das Polypeptid die Aminosäuresequenz der SEQ. ID NO:2 oder einer Isoform davon aufweist oder das Polypeptid die Aminosäuresequenz SEQ ID NO:2 umfasst,
wobei der Krebs ausgewählt ist aus der Gruppe bestehend aus Lungen-, Magen-, Speiseröhren-, Dickdarm-, Eierstock-, Hoden-, Prostata-, Mund-, Blase-, Leber-, Nieren-, Gehirn-, Blut-, Vulva-, Vagina-, Gallenblase-, Augen- und Knochenkrebs.

7. Verfahren nach Anspruch 6, umfassend die Schritte:
- Kontaktieren der biologischen Probe mit einem Antikörper oder einem Fragment davon mit einer Spezifität für ein Polypeptid mit der Aminosäuresequenz SEQ ID NO:2 oder einer Isoform davon oder einem Polypeptid umfassend die Aminosäuresequenz SEQ ID NO:2, und
- Nachweis des Bindens des Antikörpers oder Fragments davon mit einem Polypeptid mit der Aminosäuresequenz SEQ ID NO:2 oder einer Isoform davon oder einem Polypeptid umfassend die Aminosäuresequenz SEQ ID NO:2 mittels eines detektierbaren Signals, welches auf Krebs hinweist.

8. Verfahren nach Anspruch 7, wobei der Antikörper ein monoklonaler Antikörper, ein polyklonaler Antikörper, ein menschlicher oder ein rekombinanter Antikörper ist.

9. Verfahren nach Anspruch 7, wobei der Antikörper ein markierter Antikörper ist.

10. Verfahren nach einem der Ansprüche 6 bis 9, wobei die biologische Probe ein vermutetes kanzerogenes Gewebe ist, das von einer Testperson erhalten wird, bei der Krebs vermutet wird.

11. Verfahren nach einem der Ansprüche 1 bis 10, wobei die Testperson ein Säuger ist.

## Revendications

1. Procédé de détection d'un cancer chez un sujet souffrant ou suspecté de souffrir d'un cancer, comprenant l'étape de détection de la présence, dans un échantillon biologique obtenu à partir d'un sujet suspecté d'un cancer, d'un anticorps généré contre un polypeptide ayant la séquence d'acides aminés de SEQ ID NO : 2 ou une isoforme de celui-ci ou d'un anticorps généré contre un polypeptide comprenant la séquence d'acides aminés de SEQ ID NO : 2, la présences desdits anticorps indiquant un cancer,
dans lequel ledit cancer est choisi dans le groupe constitué par un cancer du poumon, de l'estomac, de l'oesophage, du côlon, ovarien, du testicule, de la prostate, oral, de la vessie, du foie, du rein, du cerveau, du sang, de la vulve, du vagin, de la vésicule biliaire, de l'oeil et des os.

2. Procédé selon la revendication 1, comprenant les étapes suivantes :
- la mise en contact dudit échantillon biologique avec un polypeptide ayant la séquence d'acides aminés de SEQ ID NO : 2 ou une isoforme de celui-ci ou un polypeptide comprenant la séquence d'acides aminés de SEQ ID NO : 2,
- la détermination de la liaison dudit polypeptide ayant la séquence d'acides aminés de SEQ ID NO : 2 ou de ladite isoforme de celui-ci ou dudit polypeptide comprenant la séquence d'acides aminés de SEQ ID NO : 2 avec un anticorps par l'intermédiaire d'un signal détectable qui indique un cancer.

3. Procédé selon la revendication 2, dans lequel ledit polypeptide ayant la séquence d'acides aminés de SEQ ID NO : 2 ou ladite isoforme de celui-ci ou ledit polypeptide comprenant la séquence d'acides aminés de SEQ ID NO : 2 est couplé à une matrice solide.

4. Procédé selon la revendication 3, dans lequel ladite matrice solide comprend du papier de nitrocellulose, une lame de verre, des plaques ou des puits de microtitrage.

5. Procédé selon l'une quelconque des revendications 1 à 4, dans lequel l'échantillon biologique est du sérum obtenu à partir d'un sujet suspecté d'un cancer

6. Procédé de détection d'un cancer chez un sujet souffrant ou suspecté de souffrir d'un cancer, comprenant l'étape de détection, dans un échantillon biologique obtenu à partir d'un sujet suspecté d'un cancer, de la présence d'un polypeptide ayant la séquence d'acides aminés de SEQ ID NO : 2 ou d'une isoforme de celui-ci ou d'un polypeptide comprenant la séquence d'acides aminés de SEQ ID NO : 2,
dans lequel ledit cancer est choisi dans le groupe constitué par un cancer du poumon, de l'estomac, de l'oesophage, du côlon, ovarien, du testicule, de la prostate, oral, de la vessie, du foie, du rein, du cerveau, du sang, de la vulve, du vagin, de la vésicule biliaire, de l'oeil et des os.

7. Procédé selon la revendication 6, comprenant les étapes suivantes :
- la mise en contact dudit échantillon biologique avec un anticorps ou un fragment de celui-ci présentant une spécificité pour un polypeptide ayant la séquence d'acides aminés de SEQ ID NO : 2 ou une isoforme de celui-ci ou un polypeptide comprenant la séquence d'acides aminés de SEQ ID NO : 2, et
- la détermination de la liaison dudit anticorps ou fragment de celui-ci avec un polypeptide ayant la séquence d'acides aminés de SEQ ID NO : 2 ou une isoforme de celui-ci ou un polypeptide comprenant la séquence d'acides aminés de SEQ ID NO : 2 par l'intermédiaire d'un signal détectable qui indique un cancer.

8. Procédé selon la revendication 7, dans lequel ledit anticorps est un anticorps monoclonal, un anticorps polyclonal, un anticorps humanisé ou recombinant.

9. Procédé selon la revendication 7, dans lequel ledit anticorps est un anticorps marqué.

10. Procédé selon l'une quelconque des revendications 6 à 9, dans lequel l'échantillon biologique est un tissu cancéreux suspecté obtenu à partir d'un sujet suspecté d'un cancer.

11. Procédé selon l'une quelconque des revendications 1 à 10, dans lequel le sujet est un mammifère.
